# EUROPÄISCHE PATENTSCHRIFT

(11) **EP 0 337 466 B1**
(45) Veröffentlichungstag und Bekanntmachung des Hinweises auf die Patenterteilung: **26.01.1994**
(21) Anmeldenummer: 89106643.3
(22) Anmeldetag: 13.04.1989
(51) Int. Cl.: G01N 33/78

(54) **Verfahren zur Bestimmung der Thyroxinbindekapazität und dazu geeignete Standardlösung**
Process for determinating the thyroxin binding capacity and standard solution therefor
Procédé pour la détermination de la capacité de liaison de thyroxine et solution standard pour sa mise en oeuvre

(30) Priorität: 15.04.1988 DE 3812610
(43) Veröffentlichungstag der Anmeldung: 18.10.1989
(73) Patentinhaber: BOEHRINGER MANNHEIM GMBH, 68298 Mannheim (DE)
(72) Erfinder: Bienhaus, Gerhard, Dr., D-8031 Wörthsee (DE); Jering, Helmut, Dr., D-8132 Tutzing (DE)
(74) Vertreter: Huber, Bernhard, Dipl.-Chem.

(56) Entgegenhaltungen:
- EP-A- 0 006 998
- EP-A- 0 298 708
- US-A- 4 052 504
- CLINICAL CHEMICAL, Band 32, Nr. 5, 1986; H.R. SCHROEDER et al., Seiten 826-830#

## Beschreibung

Die Erfindung betrifft ein Verfahren zur Bestimmung der Thyroxinbindekapazität im Serum.

Bei der Bestimmung der Thyroxinbindekapazität, die auch als "Uptake-Test" bezeichnet wird, wird die Restbindekapazität von Trägerproteinen für Schilddrüsenhormone in Patientenseren bestimmt. Die Schilddrüsenhormone liegen im Blut weitgehend in proteingebundener Form vor. Das wichtigste Trägerprotein ist dabei das Thyroxinbindende Globulin, das als TBG bezeichnet wird und an das ca. 80 % des gesamten im Blut vorliegenden Thyroxins gebunden sind. Etwa 5 bis 10 % des gesamten Thyroxins liegen an Albumin gebunden vor und etwa 10 bis 15 % des gesamten Thyroxins liegen an Präalbumin gebunden vor. Die Gesamtheit dieser Proteine wird auch als Thyroxinbindendes Protein (TBP) bezeichnet. Nur ein sehr kleiner Anteil des Thyroxins liegt im Blut in freier Form vor, in der Regel sind es ca. 0,03 %. Allein das freie Thyroxin ist physiologisch wirksam, während das gebundene Thyroxin als metabolisch inerte Transportform im Blut zirkuliert und als Puffer zur Regelung des Spiegels an freiem Thyroxin dient. Es ist daher wichtig, nicht nur den Gesamtthyroxingehalt und den Anteil an freiem Thyroxin im Blut zu bestimmen, sondern auch, die Transportkapazität des TBP zu bestimmen, da nur in Verbindung mit diesem Wert eine Diagnose im Hinblick auf die Funktion der Schilddrüse gestellt werden kann.

Zur Bestimmung der Restbindekapazität des TBP sind verschiedene Verfahren nach dem Prinzip des Immunoassays bekannt.

Bei einem aus EP-0 006 998 bekannten Verfahren werden der zu untersuchenden Serumprobe markiertes Thyroxin (T₄), immobilisierte Anti-T₄-Antikörper und eine bekannte Menge an Thyroxin zugegeben, die höher ist als die Menge, die von dem TBP noch gebunden werden kann. Dabei konkurrieren dann nichtgebundenes zugegebenes Thyroxin und markiertes Thyroxin um die Anti-T₄-Antikörper. Aus der an die Antikörper gebundenen Menge an markiertem T₄ kann man dann auf die an das TBP gebundene Menge an Thyroxin schließen und so in indirekter Weise die Bindungskapazität bestimmen.

Bei einem weiteren bekannten Verfahren, wie es in EP-0 108 400 beschrieben ist, wird in homogener Phase fluoreszenzmarkiertes T₄, das an TBP binden kann, zugegeben. Die durch diese Bindung erfolgte Änderung der Fluoreszenzpolarisation ist dann ein Maß für die Thyroxinbindekapazität.

Bei einem anderen Verfahren zur Bestimmung der Thyroxinbindekapazität, das EP-0 006 998 zu entnehmen ist, versetzt man eine bestimmte Menge von radioaktivem Trijodthyronin (T₃) mit der Serumprobe und einem Anionenaustauscher. Die freien Bindungsstellen des Trägerproteins und der Anionenaustauscher konkurrieren um das radioaktive T₃. Je mehr unbesetzte Bindungsstellen an den Trägerproteinen vorhanden sind, desto mehr radioaktives T₃ wird dort gebunden, der Rest geht an den Ionenaustauscher. Die Auswertung erfolgt, indem man entweder die Radioaktivität am Ionenaustauscher oder in der Lösung mißt. Das Verhältnis der Radioaktivität in der Lösung, die derjenigen Menge an radioaktivem T₃ entspricht, die an die Trägerproteine des zu untersuchenden Serums gebunden ist, zur Radioaktivität in der Lösung einer Standardprobe, multipliziert mit einem für den Standard spezifischen Faktor, ergibt den sogenannten Thyroxinbindungsindex TBI.

Eine weitere Möglichkeit wird in DE-OS 35 04 406 und DE-OS 35 46 014 beschrieben. Hierbei wird der Probelösung ein Unterschuß an markiertem T₄ oder T₃ und immobilisiertem Anti-T₄- bzw. -TBG-Antikörper zugegeben. Die Differenz zwischen der Menge an zugegebenem markiertem T₃ und T₄ und an der Festphase gebundenem markiertem T₃ und T₄ gibt dann die Bindekapazität von TBP wieder.

EP-A-0 298 708 gëhort zum Stand der Technik gemäß Artikel 54(3) EPÜ und beschreibt ein Verfahren zur Bestimmung des T₄-Aufnahme, das als Referenzserum ein normales humanes Serum mit unterschiedlichen TBG- und T₄-Gehalten verwendet.

Für alle diese Verfahren ist es notwendig, zur Eichung Standardlösungen mit bekannter Bindekapazität bereitzustellen. Hierbei tritt nun ein Problem auf, da einerseits die Standardlösung im wesentlichen dieselbe Zusammensetzung haben sollte wie die zu messende Lösung - also das Serum -, andererseits in der Standardlösung nur eine bekannte Menge an TBP vorhanden sein darf. Dabei ist es zur Eichung notwendig, mindestens zwei Standardlösungen mit verschiedenen Thyroxinbindekapazitäten zur Verfügung zu stellen. Zur Herstellung von Standardlösungen kann beispielsweise nach bekannten Verfahren Serum TBG-frei gemacht und dann mit gereinigtem TBG aufgestockt werden. Dieses Verfahren ist sehr aufwendig und hat außerdem den Nachteil, daS die Vermischung von zwei verschiedenen Standardlösungen zur Erzeugung von Zwischenstandardlösungen keine lineare Eichgerade ergibt, so daß es nicht möglich ist, eine Eichkurve durch Zweipunktstandardisierung zu erstellen.

Es war daher Aufgabe der vorliegenden Erfindung, Standardlösungen für die Bestimmung der Bindekapazität des TBP zur Verfügung zu stellen, die einfach herzustellen sind, gute Wiederfindungswerte ergeben und eine lineare Eichkurve ermöglichen, so daß für die Standardisierung zwei Messungen ausreichend sind.

Diese Aufgabe wird gelöst durch ein Verfahren zur Bestimmung der Thyroxinbindekapazität im Serum, das dadurch gekennzeichnet ist, daß man zur Eichung mindestens zwei verschiedene Standardlösungen verwendet, die im wesentlichen dieselbe Menge an TBG enthalten, jedoch einen unterschiedlichen Thyroxingehalt aufweisen.

Überraschenderweise wurde festgestellt, daß man sehr genaue Werte erhält, wenn man als Standardlösungen Lösungen verwendet, in denen der TBG-Gehalt konstant ist, in denen jedoch der Thyroxin-Gehalt unterschiedlich ist. Während also bisher Lösungsmöglichkeiten zur Erstellung von Standardlösungen ausschließlich in einer Variation des TBG-Gehalts der Lösungen gesucht wurden, hat sich nunmehr herausgestellt, daß eine Variation des Thyroxingehalts genauere und linearere Kurven ergibt.

Unter Thyroxin ist im Sinne der Erfindung auch Trijodthyronin zu verstehen.

Die Herstellung der Standardlösungen kann nun erfolgen ausgehend von Humanserum, wobei Serum eingesetzt wird, das thyroxinfrei ist. Thyroxinfreies Humanserum kann erhalten werden, indem man das Serum ausreichend lange mit bevorzugt immobilisierten Anti-Thyroxin-Antikörpern in Kontakt bringt. Diese thyroxinfreien Lösungen können dann jeweils mit einer bestimmten Menge an TBG versetzt werden, wobei eine thyroxinfreie Lösung als eine erste Standardlösung verwendet werden kann und eine mit Thyroxin aufgestockte als zweite.

Ebenso möglich ist es jedoch auch, Standardlösungen einzusetzen, die TBG, Albumin und ein Puffersystem enthalten. Überraschenderweise wurde festgestellt, daß derartige synthetisch hergestellte Standardlösungen ebenfalls hervorragende Wiederfindungswerte ergeben. Diese Lösungen sind leicht herzustellen und aufgrund der definierten Komponenten, die sie enthalten, sehr stabil und können daher auch über längere Zeit gelagert werden.

Die bevorzugt für das erfindungsgemäße Verfahren verwendeten Standardlösungen enthalten als wesentlichen Bestandteil Thyroxin-bindendes Globulin (TBG) Das TBG kann aus Humanseren in an sich bekannter Weise gewonnen werden. Bevorzugt enthalten die erfindungsgemäß verwendeten Standardlösungen 10 bis 80 µg/ml TBG, besonders bevorzugt 30 bis 40 µg/ml.

Bevorzugt enthalten die erfindungsgemäß verwendeten Standardlösungen weiterhin Albumin. Albumin stabilisiert die Lösungen und ermöglicht damit längere Lagerzeiten. Dazu ist es nicht erforderlich, als Albumin Humanserum-Albumin zu verwenden, obwohl dieses natürlich auch geeignet ist. Ebenso möglich ist es, die überall leicht erhältlichen und wesentlich günstigeren Albumine beispielsweise aus Rinder- und Pferdeserum zu verwenden. Bevorzugt wird als Albumin Humanserum-, Rinderserum- oder Pferdeserum-Albumin, insbesondere Rinderserumalbumin, verwendet. Das Albumin wird bevorzugt in einer Menge von 40 bis 80 mg/ml verwendet und besonders bevorzugt in physiologischer Menge eingesetzt.

Die Komponenten TBG und gegebenenfalls Albumin werden in einem Puffersystem gelöst. Als Puffersystem sind alle Puffer geeignet, die einen pH-Bereich von 6 bis 8, bevorzugt von 6,5 bis 7,5, haben, besonders bevorzugt solche mit einem pH-Wert von 7,0. Bevorzugt werden Phosphat-Puffer und GOOD-Puffer eingesetzt, beispielsweise HEPES- oder TRIS-Puffer. Die Pufferkonzentration ist nicht kritisch, bevorzugt wird der Puffer in einer Konzentration von 30 bis 150 mM/l verwendet. Besonders bevorzugt enthalten die Standardlösungen 50 bis 100 mM/l Puffer.

Die durch Auflösen von TBG und gegebenenfalls Albumin in Puffer hergestellte Lösung kann dann als erste Standardlösung verwendet werden. Zur Herstellung weiterer Standardlösungen mit unterschiedlichem Thyroxingehalt kann dann dieser Lösung das Thyroxin in den geeigneten Konzentrationen zugesetzt. In der Regel wird bei Verwendung von zwei Standardlösungen zur Herstellung einer Eichkurve eine Lösung mit geringer Konzentration an Thyroxin und eine Lösung mit hoher Konzentration an Thyroxin verwendet, wobei dann in der ersten Lösung ein hoher Prozentsatz an freien Bindungsstellen des TBPs für die Bindung des Thyroxins zur Verfügung steht, während in letzterer Lösung nur ein geringer Prozentsatz an Bindungsstellen für das Thyroxin vorhanden ist. Bevorzugt enthält die Lösung mit hoher Bindekapazität bis zu 25 ng/ml Thyroxin. Sie kann auch Thyroxin-frei sein. Die Lösung mit geringer Bindekapazität enthält bevorzugt 250 bis 500 ng/ml Thyroxin, besonders bevorzugt 300 bis 350 ng/ml. Dabei stellt sich dann ein Gleichgewicht zwischen proteingebundenem und freiem Thyroxin ein analog dem in vivo im Blut herrschenden Gleichgewicht. Diese Lösungen sind aufgrund ihrer Zusammensetzung auch bei längerer Lagerung stabil. Da sie aus standardisierten Einzelsubstanzen hergestellt wurden, weisen sie eine stets gleichmäßige Zusammensetzung auf und liefern daher reproduzierbare Werte.

Ein weiterer Gegenstand der Erfindung sind Standardlösungen für die Bestimmung der Thyroxinbindekapazität, die TBG gelöst in einem Puffersystem enthalten.

Die Standardlösungen unterscheiden sich durch den Gehalt an Thyroxin. Der Thyroxingehalt der Lösung wird durch Zugabe einer bestimmten Menge Thyroxin eingestellt.

Bevorzugt enthält die Standardlösung 10 bis 80 µg/ml TBG, insbesondere 20 bis 50 µg/ml TBG und besonders bevorzugt physiologische Mengen an TBG.

Das Puffersystem weist geeigneterweise einen pH im Bereich von 6 bis 8, vorzugsweise von 6,5 bis 7,5 auf und ist bevorzugt ein GOOD-Puffer oder Phosphatpuffer.

Bevorzugt enthält die Standardlösung zur Erhöhung der Stabilität weiterhin noch Albumin, bevorzugt im Bereich von 40 bis 80 mg/ml Albumin.

Erfindungsgemäß wird weiterhin eine Standardkombination für die Bestimmung der Thyroxinbindekapazität zur Verfügung gestellt, die aus zwei physikalisch voneinander getrennten Lösungen besteht, von denen die eine 10 bis 80 µg/ml TBG und 20 bis 800 ng/ml Thyroxin gelöst in einem Puffersystem und die andere 10 bis 80 µg/ml TBG gelöst in einem Puffersystem enthält.

Die Standardlösungen werden beispielsweise hergestellt, indem man physiologische Mengen an TBG und gegebenenfalls Albumin in einem Puffersystem löst und die jeweils gewünschte Menge an Thyroxin zugibt.

Erfindungsgemäß werden Standardlösungen mit überlegener Stabilität zur Verfügung gestellt, die in einfacher Weise aus leicht erhältlichen Ausgangsmaterialien hergestellt werden können. Darüber hinaus ergeben die mit den erfindungsgemäßen Standardlösungen erhaltenen Eichwerte lineare Kurven, so daß die Bestimmung zweier Standardwerte für die Standardisierung der Bestimmung ausreicht.

Die Erfindung wird durch die folgenden Figuren und Beispiele erläutert.
- Figur 1: zeigt eine Eichkurve, die mit Standardlösungen nach dem Stand der Technik für die Bestimmung der Thyroxinbindekapazität erstellt wurde;
- Figur 2: zeigt eine Eichkurve, die mit erfindungsgemäßen Standardlösungen auf Basis von Humanserum mit verschiedenen T₄-Konzentrationen erstellt wurde.
- Figur 3: zeigt eine Eichkurve, die mit erfindungsgemäßen Standardlösungen auf Basis einer Pufferlösung mit verschiedenen T₄-Konzentrationen erstellt wurde.

### Beispiel 1

Es wurden Standardlösungen, wie sie im Stand der Technik beschrieben sind, hergestellt.
- Standardlösung 1: enthält TBG-freies Serum, aufgestockt mit 3,75 µg TBG/ml;
- Standardlösung 2: enthält TBG-freies Serum, aufgestockt mit 150 µg TBG/ml.

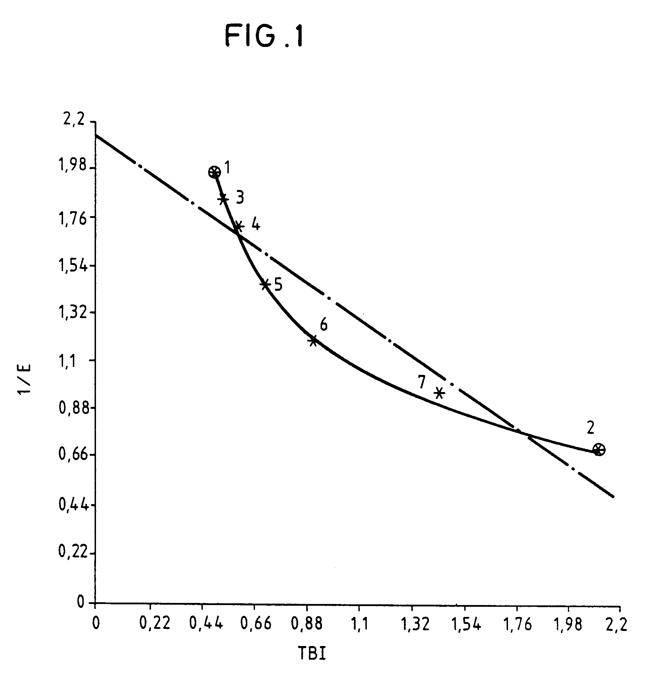

Mit diesen beiden Standardlösungen wurden weitere Zwischenstandards 3 bis 7 (vgl. Fig. 1) hergestellt:

| Zwischenstandard | Stand.lösg. 1 Vol.-Teile | Stand.lösg. 2 Vol.-Teile |
|---|---|---|
| 3 | 9 | 1 |
| 4 | 8 | 2 |
| 5 | 6 | 4 |
| 6 | 4 | 6 |
| 7 | 2 | 8 |

Die TBG-Bestimmung erfolgte, wie in Beispiel 2 beschrieben. Zur Auswertung wurde die reziproke Extinktion (1/E) gegen den Thyroxinbindungsindex (TBI) aufgetragen.

### Beispiel 2

Es wurde eine erfindungsgemäße Standardlösung auf der Basis einer Humanserummatrix hergestellt. Dazu wurden an BrCN-Sephadex gereinigte Antikörper gegen Thyroxin vom Schaf, die nach der Methode von K. Heide et al (1973), "Handbook of Experimental Immunology", Bd. 1, 2. Aufl., D.M. Weir, Hrsg., Oxford Blackwell Scientific, 6.1-7.16, erhalten wurden, nach der Methode von Fuchs et al 1973), "Handbook of Experimental Immunology", Bd. 1, 2. Aufl., D.M. Weir, Hrsg., wie oben, 11.1-11.4, gekoppelt. Auf diese Weise wurde ein Immunadsorber mit ausreichender Thyroxinbindekapazität erhalten. 6 l sterilfiltriertes Humanserum wurden dann bei 37 °C über den Immunadsorber gepumpt, der zuvor mit 0,9 %iger NaCl-Lösung equilibriert worden war. Anschließend wurde mit 1 l von 0,9 %iger NaCl-Lösung nachgewaschen. Das Eluat wurde direkt in eisgekühlten Vorlagen aufgefangen, gepoolt und sterilfiltriert. Der Thyroxingehalt der erhaltenen Lösung wurde mit Hilfe eines Enzymimmunoassays bestimmt. Er war geringer als 20 ng T₄/ml.

Die so erhaltene, im wesentlichen Thyroxin-freie Humanserumlösung wurde dann mit TBG aufgestockt. Dazu wurde lyophylisiertes TBG verwendet, das hergestellt wurde, wie in B. Kagedahl et al (1977), Clinica Chimica Acta 78, 103-111, beschrieben. Das TBG wurde mit einer Konzentration von 1 mg TBG/ml in dem T₄-armen Humanserum gelöst. Mit dieser TBG-Lösung wurde durch Vermischen mit dem T₄-armen Humanserum eine Verdünnungsreihe hergestellt mit Lösungen, die 0; 5; 10; 20; 40; 50; 100 mg TBG/l enthielten. In diesen Lösungen wurde, wie in EP-0 006 998 beschrieben, mit einem Enzymimmunoassay der Thyroxinbindeindex bestimmt. Hierzu wurden in mit Anti-T₄-Antikörper beschichtete Reagenzgläschen 10 µl Serum und anschließend 1 ml eines Reagenzes pipettiert, das 280 ng T₄/ml und 10 mU/ml T₄-POD in 0,12 M-Barbitalpuffer, pH 8,6, und 0,2 Rinderserumalbumin enthielt. Dann wurde zwei Stunden bei Raumtemperatur stehen gelassen. Danach wurden die Gläschen durch Aussaugen entleert und das POD-Reagenz eingefüllt. Letzteres bestand aus 1,47 mM/l H₂O₂ und 14 mM/l ABTS (2,2′-Azino-di- 3-ethyl-benzthiazolin-sulfonsäure(6) -diammoniumsalz) in 0,2 M-Phosphatcitratpuffer, pH 5,0. Die auftretende Farbänderung wurde bei 405 nm gemessen.

Aus den erhaltenen Werten ergibt sich die Menge an TBG, mit der das T₄-arme Humanserum aufgestockt werden muß, um ein Meßsignal zu erzeugen, das dem gewünschten Wert für die höchste Thyroxinbindekapazität entspricht. Im vorliegenden Beispiel war diese Menge 15 mg TBG/l, was einen Thyroxinbindeindexwert (TBI-Wert) von 1,39 ergab.

Der so hergestellten Lösung (Standardlösung 2), die eine bekannte Menge an TBG enthielt, wurde L-Thyroxin in verschiedenen Konzentrationen zugesetzt. Dazu wurde L-Thyroxin in wenig NaOH und Ethanol gelöst und in T₄-armem Humanserum zu einer Lösung mit einer Konzentration von 1 mg T₄/ml verdünnt. Aus dieser Lösung wurde eine Verdünnungsreihe hergestellt, wobei Lösungen erhalten wurden, die 0; 300; 400; 500; 600 ng T₄/ml enthielten. Die Thyroxinbindekapazität dieser Lösungen wurde wiederum bestimmt. Diejenige Lösung, deren Meßsignal der gewünschten niedrigen Thyroxinbindekapazität entsprach, wurde als Standardlösung weiterverwendet. Das so aufgestockte Serum wurde eine Stunde bei Raumtemperatur gerührt, um eine vollständige Gleichgewichtseinstellung zu bewirken.

Im vorliegenden Beispiel erwies sich die Standardlösung mit 420 ng T₄/ml geeignet als Standardlösung 2. Dies entspricht einem TBI-Wert von 0,11.

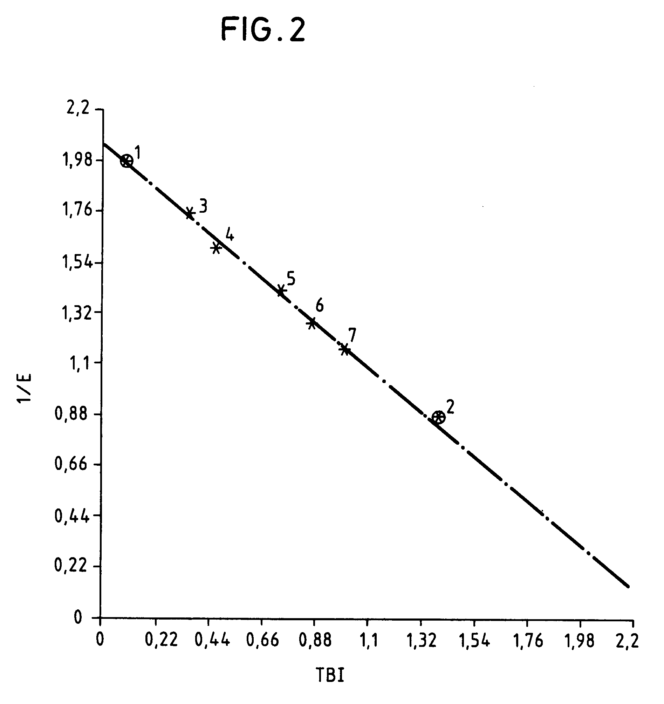

Mit den so hergestellten Standardlösungen wurde nun eine Eichkurve erstellt. Dazu wurden verschiedene Mischungen der beiden Standardlösungen 1 und 2 hergestellt und wie in Fig. 2 mit 3 bis 7 bezeichnet. Diese Standardlösungen wurden folgendermaßen hergestellt:

| Standard | Vol.-Teile 1 | Vol.-Teile 2 |
|---|---|---|
| 3 | 8 | 2 |
| 4 | 7 | 3 |
| 5 | 5 | 5 |
| 6 | 4 | 6 |
| 7 | 3 | 7 |

Von den Lösungen wurde dann, wie oben beschrieben, die Thyroxinbindekapazität bestimmt. Den Mischungen wurden jeweils die rechnerisch sich ergebenden TBI-Werte zugeordnet. Diese rechnerischen TBI-Werte wurden dann gegen die reziproken Meßwerte der Extinktion (1/E) aufgetragen. Die Ergebnisse sind der Fig. 2 zu entnehmen.

Die Korrelationskoeffizienten einer linearen Regressionsanalyse waren:
- R = 0,9989: für die erfindungsgemäßen Standardlösungen (Fig. 2) und
- R = 0,9349: für Standardlösungen nach dem Stand der Technik (Fig. 1).

### Beispiel 3

Es wurde eine Standardlösung hergestellt. Dazu wurde lyophylisiertes TBG, das, wie in Beispiel 2 beschrieben, hergestellt worden war, in Phosphatpuffer, der aus 50 mM/l NaH₂PO₄; 60 mg/ml Rinderserumalbumin; 0,1 % Tween 20; 0,9 NaCl, mit NaOH auf pH 7,4 eingestellt, bestand, gelöst mit einer Konzentration von 1 mg TBG/ml. Aus dieser Lösung wurde eine Verdünnungsreihe mit Phosphatpuffer hergestellt. Für die einzelnen Lösungen wurde, wie in Beispiel 2 beschrieben, der Thyroxinbindekapazitätswert (TBK-Wert) bestimmt. Standardlösung 2 enthielt 48 mg TBG/l. Dies ergab ein Meßsignal, das einem TBI-Serumwert von 1,43 entspricht.

Es wurde, wie in Beispiel 2 beschrieben, eine T₄-Lösung hergestellt, wobei L-Thyroxin statt in Humanserum in Phosphatpuffer gelöst wurde. Die Verdünnungsreihe wurde mit Standardlösung 2 hergestellt. Es wurde sodann, wie in Beispiel 2 beschrieben, von den verschiedenen Lösungen der TBK-Wert bestimmt. Als Standardlösung 1 wurde eine Lösung, die 420 ng T₄/ml enthielt, verwendet. Dieser Standardlösung konnte ein TBK-Wert von 0,32 zugeordnet werden.

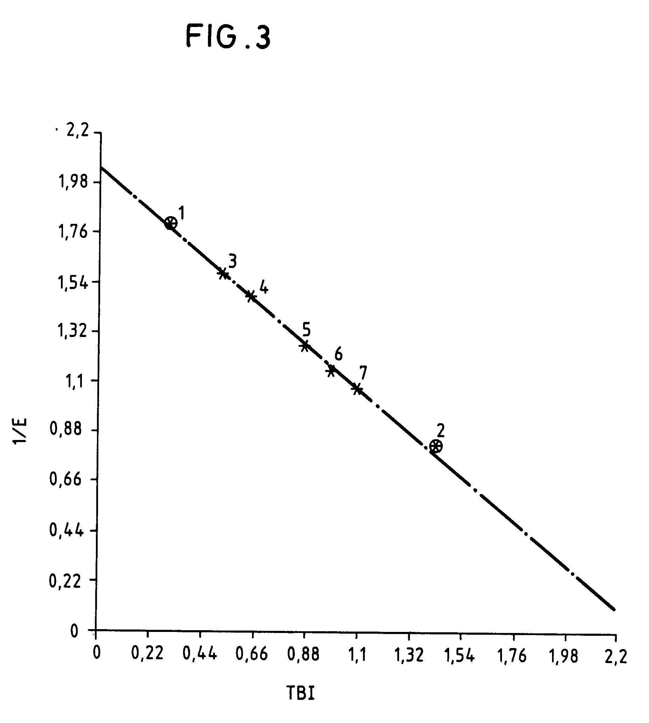

Von den beiden Standardlösungen 1 und 2 wurden wiederum Mischungen (in Fig. 3 mit 3 bis 7 bezeichnet) hergestellt und von diesen Mischungen die TBK-Werte bestimmt.

Die Standardlösungen 3 bis 7 wurden folgendermaßen hergestellt:

| Standard | Vol.-Teile 1 | Vol.-Teile 2 |
|---|---|---|
| 3 | 8 | 2 |
| 4 | 7 | 3 |
| 5 | 5 | 5 |
| 6 | 4 | 6 |
| 7 | 3 | 7 |

Die Thyroxinbindekapazität wurde wie in Beispiel 2 bestimmt und die reziproke Extinktion (1/E) gegen TBI aufgetragen.

Es ergab sich wiederum eine lineare Kurve, deren Korrelationskoeffizient der linearen Regressionsanalyse 0,9974 ergab (Fig. 3).

## Patentansprüche

1. Verfahren zur Bestimmung der Thyroxinbindekapazität im Serum,
**dadurch gekennzeichnet,**
daß man zur Eichung mindestens zwei verschiedene Standardlösungen verwendet, die im wesentlichen dieselbe Menge an TBG (Thyroxine bindende Globulin) enthalten, jedoch einen unterschiedlichen Thyroxingehalt aufweisen.

2. Verfahren nach Anspruch 1,
**dadurch gekennzeichnet,**
daß eine der verwendeten Standardlösungen kein Thyroxin enthält.

3. Verfahren nach Anspruch 1,
**dadurch gekennzeichnet,**
daß man Standardlösungen verwendet, die physiologische Mengen an TBG gelöst in einem Puffersystem sowie Thyroxin in bestimmter Menge enthalten.

4. Verfahren nach einem der vorhergehenden Ansprüche,
**dadurch gekennzeichnet,**
daß die Standardlösungen zusätzlich Albumin enthalten.

5. Verfahren nach Anspruch 4,
**dadurch gekennzeichnet,**
daß man Standardlösungen verwendet, die 40 bis 80 mg/ml Albumin enthalten.

6. Verfahren nach einem der vorhergehenden Ansprüche,
**dadurch gekennzeichnet,**
daß man Standardlösungen verwendet, die physiologische Mengen Albumin enthalten.

7. Verfahren nach einem der Ansprüche 1 oder 3 bis 6,
**dadurch gekennzeichnet,**
daß man Standardlösungen verwendet, die 20 bis 800 ng/ml Thyroxin enthalten.

8. Verfahren nach einem der vorhergehenden Ansprüche,
**dadurch gekennzeichnet,**
daß man Standardlösungen verwendet, die 10 bis 80 µg/ml TBG enthalten.

9. Standardkombination für die Bestimmung von der Thyroxinbindekapazität in Serum, bestehend aus zwei physikalisch voneinander getrennten Lösungen, von denen die eine 10 bis 80 µg/ml TBG (Thyroxine bindende Globulin) und 20 bis 800 ng/ml Thyroxin gelöst in einem Puffersystem und die andere 10 bis 80 µg/ml TBG gelöst in einem Puffersystem enthält, wobei beide Lösungen die gleiche TBG-Konzentration aufweisen.

## Claims

1. Process for the determination of the thyroxine-binding capacity in serum, characterised in that, for the calibration, one uses at least two different standard solutions which contain substantially the same amount of TBG (thyroxine-binding globulin) but a differing thyroxine content.

2. Process according to claim 1, characterised in that one of the standard solutions used contains no thyroxine.

3. Process according to claim 1, characterised in that one uses standard solutions which contain physiological amounts of TBG dissolved in a buffer system, as well as thyroxine in definite amount.

4. Process according to one of the preceding claims, characterised in that the standard solutions additionally contain albumin.

5. Process according to claim 4, characterised in that one uses standard solutions which contain 40 to 80 mg/ml of albumin.

6. Process according to one of the preceding claims, characterised in that one uses standard solutions which contain physiological amounts of albumin.

7. Process according to one of claims 1 or 3 to 6, characterised in that one uses standard solutions which contain 20 to 800 ng/ml of thyroxine.

8. Process according to one of the preceding claims, characterised in that one uses standard solutions which contain 10 to 80 µg/ml of TBG.

9. Standard combination for the determination of the thyroxine binding capacity in serum, consisting of two solutions physically separated from one another, one of which contains 10 to 80 µg/ml TBG (thyroxine-binding globulin) and 20 to 800 ng/ml thyroxine dissolved in a buffer system and the other contains 10 to 80 µg/ml TBG dissolved in a buffer system, whereby both solutions have the same TBG concentration.

## Revendications

1. Procédé pour déterminer la capacité de liaison à la thyroxine dans le sérum, caractérisé en ce que, pour l'étalonnage, on utilise au moins deux solutions standard différentes qui contiennent sensiblement la même quantité de GLT (globuline liant la thyroxine) mais qui présentent des teneurs en thyroxine différentes.

2. Procédé selon la revendication 1, caractérisé en ce que l'une des solutions standard utilisées ne contient pas de thyroxine.

3. Procédé selon la revendication 1, caractérisé en ce que l'on utilise des solutions standard qui contiennent des quantités physiologiques de GLT dissoute dans un système tampon ainsi que de la thyroxine en une quantité déterminée.

4. Procédé selon l'une des revendications précédentes, caractérisé en ce que les solutions standard contiennent en outre de l'albumine.

5. Procédé selon la revendication 4, caractérisé en ce que l'on utilise des solutions standard qui contiennent 40 à 80 mg/ml d'albumine.

6. Procédé selon l'une des revendications précédentes, caractérisé en ce que l'on utilise des solutions standard qui contiennent des quantités physiologiques d'albumine.

7. Procédé selon l'une des revendications 1 ou 3 à 6, caractérisé en ce que l'on utilise des solutions standard qui contiennent 20 à 800 ng/ml de thyroxine.

8. Procédé selon l'une des revendications précédentes, caractérisé en ce que l'on utilise des solutions standard qui contiennent 10 à 80 µg/ml de GLT.

9. Combinaison standard pour la détermination de la capacité de liaison à la thyroxine dans le sérum, consistant en deux solutions physiquement séparées l'une de l'autre, parmi lesquelles l'une contient 10 à 80 µg/ml de GLT (globuline liant la thyroxine) et 20 à 800 ng/ml de thyroxine dissoute dans un système tampon et l'autre contient 10 à 80 µg/ml de GLT dissoute dans un système tampon, les deux solutions présentant la même concentration en GLT.
